# DEMANDE DE BREVET EUROPEEN

(11) **EP 0 523 316 A1**
(43) Date de publication de la demande: **20.01.1993**
(21) Numéro de dépôt: 92104158.8
(22) Date de dépôt: 11.03.1992
(51) Int. Cl.: C12N 1/20, C12G 1/02, C12H 1/00

(54) **Levain malolactique**

(30) Priorité: 16.04.1991 CH 1138/91
(71) Demandeur: SOCIETE DES PRODUITS NESTLE S.A., CH-1800 Vevey (CH)
(72) Inventeur: d'Amico, Nicola, CH-1400 Yverdon (CH); Dac, Thang Ho, CH-1052 Le Mont, Lausanne (CH); Sozzi, Tomaso, CH-1010 Lausanne (CH); Wood, Robert Dustan, CH-1004 Lausanne (CH)

(57) **Abrégé**

Levain malolactique capable de déclencher une fermentation malolactique dès son inoculation directe dans un vin normal ou léger et capable de la mener rapidement à terme.

## Description

La présente invention a pour objet un procédé de préparation d'un levain malolactique, le levain ainsi obtenu, ainsi que son utilisation dans la préparation d'un vin.

On connaît divers procédés de désacidification d'un vin par une fermentation malolactique au cours de laquelle des bactéries malolactiques, notamment Leuconostoc oenos et certains Lactobacillus, convertissent en acide lactique l'acide malique contenu dans ce vin.

US 4380552 propose par exemple de désacidifier un vin en le faisant passer au travers d'un lit de billes de gel renfermant des cellules viables de Leuconostoc oenos. Il n'est cependant pas certain que ce procédé soit meilleur qu'une inoculation directe de cette bactérie dans le vin.

EP 327380 propose de réaliser une fermentation malolactique contrôlée d'un vin en le faisant passer au travers d'un récipient contenant au moins 10⁸ cellules de bactéries malolactiques par ml et délimité en amont et en aval par des filtres retenant les bactéries. La même charge de bactéries pourrait être utilisée relativement longtemps en continu ou jusqu'à cinq ou six fois en travaillant par lots. Le procédé et l'équipement semblent cependant relativement compliqués.

US 4562077 propose de réduire la teneur en acide malique d'un vin en convertissant cet acide en acide lactique à l'aide de levains congelés ou lyophilisés contenant, à raison de 10⁸-10¹³ cellules/ml, des souches de Leuconostoc oenos sélectionnées pour leur bonne activité. Les levains proposés doivent cependant être spécialement activés avant de pouvoir être inoculés dans le vin.

US 4547373 propose des souches de Leuconostoc oenos capables de réaliser une fermentation malolactique relativement vite, à savoir en environ 50-100 d, à une température relativement basse de environ 16-18°C et à un pH relativement bas de environ 3,1-3,2 par exemple.

On peut, par ailleurs, se procurer dans le commerce des levains malolactiques destinés à être utilisés en vinification courante, lorsque l'on désire mieux contrôler une fermentation malolactique d'un moût ou d'un vin riche en acide malique. Cependant, ces levains du commerce doivent pratiquement tous être réactivés avant l'emploi, par exemple durant 2 jours sur un milieu particulier, ce qui complique le travail en cave.

On connaît également divers procédés de préparation de vins dits légers, à savoir de vins dont la teneur en alcool est sensiblement réduite par rapport à celle d'un vin dit normal qui présente une teneur en alcool de environ 8,5-16% en volume (8,5-16° d'alcool).

US 4626437 décrit par exemple un procédé de préparation d'un vin léger dans lequel on soumet un vin normal à un processus d'évaporation pour en séparer de l'alcool et des composants aromatiques, on obtient un condensat dont on sépare une fraction aromatique par distillation fractionnée et l'on retourne cette fraction aromatique au vin appauvri en alcool et en composants aromatiques. Un tel vin léger peut présenter malgré tout un arôme sensiblement modifié par rapport à celui du vin de départ.

US 4902518 décrit un procédé de préparation d'un vin léger dans lequel on sépare un jus de raisin en une fraction riche en sucre et une fraction pauvre en sucre, on récupère une fraction aromatique de la fraction riche en sucre et on l'ajoute à la fraction pauvre en sucre que l'on soumet alors à une fermentation traditionnelle.

Un vin léger ainsi obtenu peut également présenter un arôme sensiblement distinct de l'arôme d'un vin obtenu par fermentation traditionnelle du jus de raisin de départ.

La flaveur d'un vin étant sensiblement influencée, voire renforcée par une fermentation malolactique accompagnant ou suivant la fermentation alcoolique, il pourrait être avantageux de soumettre de tels vins légers à une fermentation malolactique au cours de ou à la fin de leur préparation. Une telle opération sous-entendrait cependant la maîtrise de la fermentation malolactique.

Il existe donc un besoin d'un levain malolactique sûr et efficace capable de déclencher une fermentation malolactique dès son inoculation directe dans un vin normal ou léger et capable de la mener rapidement à terme.

La présente invention a pour but de pallier les défauts des procédés de désacidification et des levains connus et de couvrir ledit besoin en levain malolactique sûr et efficace de manière à pouvoir améliorer la préparation de vins normaux ou légers.

A cet effet, dans le procédé de préparation d'un levain malolactique selon la présente invention, on prépare un milieu de culture contenant de l'alcool, une source d'azote assimilable et de l'acide malique, on prépare une biomasse par inoculation et culture d'au moins une souche de bactérie malolactique dans ledit milieu de culture, et l'on sépare ladite biomasse.

On a constaté avec surprise qu'un levain préparé de cette manière peut effectivement être inoculé directement dans un vin et y déclencher immédiatement, de manière sûre et fiable, une fermentation malolactique vigoureuse et rapide.

Pour mettre en oeuvre le présent procédé, on peut préparer ledit milieu de culture contenant de l'alcool par fermentation alcoolique d'un milieu de base contenant un sucre fermentescible, notamment un jus de fruit ou une solution aqueuse à environ 5-15% en poids de saccharose, glucose ou fructose, par exemple, ce jus ou cette solution contenant par ailleurs ladite source d'azote assimilable et l'acide malique. Pour réaliser cette fermentation alcoolique, on peut utiliser toute levure connue pour produire de l'alcool, notamment des levures utilisées en oenologie et plus particulièrement des levures de l'espèce Saccharomyces cerevisiae, par exemple. On peut inoculer environ 1-5% en volume d'un levain traditionnel ou environ 0,02-0,1% en poids d'un levain concentré de S.cerevisiae du commerce dans ledit milieu de base à température ambiante et laisser fermenter durant environ 1-4 d, par exemple. Le pH du milieu de base étant de environ 2,8-3,2 après une telle fermentation, on peut alors l'ajuster, le cas échéant, à environ 3-4,5, de préférence 3,4-3,6, par addition d'une solution concentrée de NaOH, par exemple.

On peut également préparer ledit milieu de culture contenant de l'alcool par mélange direct d'alcool et d'eau, le mélange pouvant présenter une teneur en alcool de environ 2,5-8% en volume, par exemple, et ce mélange comprenant par ailleurs ladite source d'azote assimilable et l'acide malique. Si l'on prépare ledit milieu de culture de cette manière, on peut également ajuster son pH à 3-4,5, de préférence 3,4-3,6.

Dans ledit milieu de culture, on peut utiliser comme source d'azote assimilable diverses matières riches en acides aminés ou en polypeptides telles qu'un extrait de levure, un hydrolysat de protéines végétales ou animales, une liqueur de trempage du maïs ou du citrate d'ammonium, à raison de environ 0,1-1% en poids, par exemple.

En ce qui concerne l'acide malique, on peut l'incorporer dans ledit milieu de culture à raison de environ 0,1-2% en poids, par exemple.

On peut également ajouter audit milieu de culture environ 0,05-0,5% en poids de glucose pour faciliter, le cas échéant, la croissance de ladite bactérie malolactique, et environ 1-10 mg/l de SO₂, sous forme de bisulfite de sodium, par exemple, pour induire une résistance de la bactérie malolactique au SO₂, ou plus précisément du H₂SO₃ qui se forme dans le milieu par suite de cette addition.

On peut inoculer dans ledit milieu de culture environ 0,5-5% d'une culture contenant environ 10⁷-10⁹ cellules d'au moins une souche de bactérie malolactique par ml. On peut choisir cette ou ces souches parmi des souches de bactéries lactiques capables de réaliser la fermentation malolactique et de produire une flaveur agréable telles que des souches de Leuconostoc oenos, Lactobacillus brevis ou Lactobacillus plantarum, par exemple. On choisit de préférence cette ou ces souches parmi des souches de Leuconostoc oenos que l'on peut isoler à partir de vins faisant leur fermentation malolactique dans des caves de vignerons d'Europe centrale et plus particulièrement de Suisse, par exemple. Parmi de nombreuses souches préférées ainsi isolées, on en a sélectionné quatre à partir desquelles on peut préparer un levain de qualité particulièrement remarquable. On a déposé ces quatre souches de Leuconostoc oenos, à titre d'exemple, selon le Traité de Budapest, le 29.03.91, à la Collection Nationale de Cultures de Microorganismes (CNCM), Institut Pasteur, 28 rue du Dr Roux, 7524 Paris Cedex 15, France, où elles ont reçu les Nos CNCM I-1063, I-1064, I-1065 et I-1066. Ces quatre souches sont très semblables entre elles et ne se distinguent pratiquement les unes des autres que par une sensibilité différente aux phages. Des détails concernant leur morphologie et leurs propriétés sont donnés ci-après.

### CNCM I-1064

- Morphologie:: - Microorganisme Gram positif, catalase négatif et anaérobe facultatif.
- Petites cellules sphériques, parfois lenticulaires, en chaînes plus ou moins longues (sur milieu MRS).
- Les cellules peuvent être allongées, voire même sous forme de bacilles sur vin (spécialement sur vin acide, pH 3,0-3,2).
- Pas de présence de flagelles, pas de formation de spores.
- Fermentation des sucres:: - Production d'acide à partir du glucose.
- Autres:: - Capacité à fermenter le malate en acide lactique L(+) en présence d'alcool (fermentation malolactique).

### CNCM I-1063, I-1065 et I-1066

La morphologie et les propriétés de chacune de ces souches sont les mêmes que celles décrites ci-dessus pour la souche CNCM I-1064.

On peut cultiver ladite souche de bactérie lactique dans ledit milieu de culture à température ambiante, autrement dit à une température d'environ 18-30°C, durant environ 1-4 d, jusqu'à ce que le milieu contienne environ 10⁷-10⁹ cellules/ml.

On peut ensuite séparer et, le cas échéant, concentrer environ 20-100 fois la biomasse ainsi obtenue, par ultrafiltration ou centrifugation, par exemple.

Après cette séparation et cette éventuelle concentration, on peut déshydrater la biomasse, notamment par lyophilisation, par exemple. De préférence, on la congèle après lui avoir ajouté un agent cryoprotecteur tel que le glycerol, par exemple, et après avoir ajusté son pH à environ 4,2-4,8.

Le levain malolactique ainsi obtenu peut contenir environ 5.10⁸-10¹¹ cellules/g. Il peut être utilisé pour la conversion de l'acide malique en acide lactique au cours de la préparation d'un vin. Il se distingue par sa capacité à déclencher une fermentation malolactique dès son inoculation directe dans un vin, et il est capable de mener cette fermentation rapidement à son terme.

Outre l'utilisation du présent levain dans la préparation d'un vin dit normal, la présente invention a également pour objet son utilisation dans la préparation d'un vin dit léger.

C'est ainsi que la présente invention a notamment pour objet l'utilisation du présent levain dans un premier procédé particulier de préparation d'un vin léger comprenant une séparation, notamment par ultrafiltration, d'une partie du sucre d'un moût de raisin, une fermentation alcoolique du moût appauvri en sucre et une fermentation malolactique du vin à faible teneur en alcool ainsi obtenu.

De même, la présente invention a aussi pour objet l'utilisation du présent levain dans un second procédé particulier de préparation d'un vin léger comprenant une fermentation alcoolique au moins partielle d'un moût de raisin, une élimination d'une partie au moins de l'alcool produit durant cette fermentation alcoolique, et une fermentation malolactique et/ou alcoolique secondaire du vin à faible teneur en alcool ainsi obtenu.

Pour réaliser ces utilisations, on peut inoculer directement le présent levain, à raison de environ 0,01-0,1% en poids, dans un vin à environ 18-30°C qui présente une teneur en SO₂ total inférieure à environ 50 mg/l ainsi qu'un pH adéquat dépendant de sa teneur en alcool. Pour un vin dit normal présentant une teneur en alcool de environ 8,5 à 16% en volume, ce pH adéquat peut être de environ 3,1-4,5. Pour un vin dit léger présentant une teneur en alcool de environ 0,5-8,5% en volume, ce pH adéquat peut être de environ 2,6-4,0.

Les exemples ci-après sont présentés à titre d'illustration du procédé de préparation et des utilisations du levain malolactique selon la présente invention. Les pourcentages et parties y sont donnés en poids sauf indication contraire.

### Exemple 1

On prépare un milieu de base sous forme d'une solution aqueuse à 10% de saccharose contenant en outre 0,5% d'extrait de levure, 0,5% de liqueur de trempage de maïs et 0,5% d'acide malique. On inocule dans ce milieu de base 2% en volume d'un levain de Saccharomyces cerevisiae du commerce pour vinification et l'on laisse fermenter durant 3 d à température ambiante.

On obtient ainsi un milieu de culture présentant une teneur en alcool de 6,2% en volume et un pH de 3,1 que l'on ajuste à 3,4 par addition d'une solution aqueuse à 20% de NaOH. On lui ajoute 3 mg/l de SO₂ sous forme de bisulfite de sodium et 0,1% de glucose.

On inocule dans ce milieu de culture 3% en volume d'une culture contenant environ 10⁷ cellules/ml de chacune des souches de Leuconostoc oenos CNCM I-1063, I-1064, I-1065 et I-1066. On cultive ces souches dans ce milieu à température ambiante durant 3 d. On sépare par centrifugation la biomasse de Leuconostoc oenos ainsi obtenue. On la remet en suspension dans un milieu aqueux cryoprotecteur contenant 10% de glycerol et l'on ajuste son pH à 4,5. On obtient un levain malolactique dont le volume est réduit de 50 fois par rapport au milieu de culture et qui contient environ 2.10⁹ cellules/ml.

On congèle ce levain par pulvérisation dans l'azote liquide. On obtient ainsi un levain congelé sous forme de billes prêtes à l'emploi qui contiennent donc environ 2.10⁹ cellules de Leuconostoc oenos par g.

### Exemple 2

Après fermentation alcoolique d'un moût sulfité à raison de 40 mg SO₂/l, un vin de Chasselas présente une teneur en alcool de 11,4 % en volume, un pH de 3,39, une teneur en acite tartrique de 3,3 g/l, une teneur en acide malique de 3,2 g/l et une teneur en SO₂ total de 10 mg/l.

On inocule dans ce vin, en cuve de 100 l, 0,02% en poids de billes congelées du levain malolactique obtenu à l'exemple 1. On suit l'évolution de ce vin ensemencé par rapport à celle du même vin, en cuve de 100 l, non ensemencé (pour comparaison).

On constate une diminution de la teneur en acide malique du vin ensemencé de 3,2 à 2,5 g/l au cours de la première semaine, de 2,5 à 1,2 g/l au cours de la deuxième semaine et de 1,2 g/l à pratiquement 0 g/l entre la quatorzième et le vingt-troisième d.

Par contre, il faut environ 40 d pour que la teneur en acide malique du vin non ensemencé tombe à pratiquement 0 g/l, le départ réel de la fermentation malolactique étant différé de près de 20 d par rapport à celle du vin ensemencé.

En fin de fermentation malolactique, donc après respectivement 23 d et 40 d, le vin ensemencé et le vin non ensemencé contiennent chacun plus de 10⁷ cellules de Leuconostoc oenos par ml. Ils sont aussi bons l'un que l'autre.

### Exemple 3

On sulfite un moût de Gamay à raison de 75 mg SO₂/l. On déclenche sa fermentation alcoolique 5 h plus tard en lui inoculant 0,04% en poids d'une suspension réactivée de levain de Saccharomyces cerevisiae déshydraté du commerce. On laisse fermenter à 21-29°C durant 2-3 d. On interrompt la fermentation lorsque la teneur en alcool atteint 7% en volume.

On sépare la pulpe pour pressage et l'on soumet le vin partiellement fermenté ainsi obtenu à une distillation sous vide au cours de laquelle on récupère tout d'abord des arômes volatils puis on réduit sa teneur en alcool à environ 4-5% en volume. On retourne ensuite les arômes récupérés au vin à faible teneur en alcool ainsi obtenu. Ce vin présente par ailleurs un pH de 3,23 et une teneur en SO₂ total négligeable.

On refroidit ce vin à 18°C et on lui inocule 1% en volume d'un levain de Saccharomyces cerevisiae du comerce et 0,1% en poids de billes congelées du levain de Leuconostoc oenos obtenu à l'exemple 1. On laisse se dérouler simultanément la fermentation malolactique et la fermentation alcoolique secondaire durant 12 d à 18-20°C.

On obtient ainsi un vin léger qui présente une teneur en alcool de 8,2% en volume, une teneur pratiquement nulle en acide malique résiduel et une flaveur de vin agréable.

## Revendications

1. Procédé de préparation d'un levain malolactique, dans lequel on prépare un milieu de culture contenant de l'alcool, une source d'azote assimilable et de l'acide malique, on prépare une biomasse par inoculation et culture d'au moins une souche de bactérie malolactique dans ledit milieu de culture, et l'on sépare ladite biomasse.

2. Procédé selon la revendication 1, dans lequel on prépare ledit milieu de culture contenant de l'alcool par fermentation alcoolique d'un milieu de base contenant un sucre fermentescible.

3. Procédé selon la revendication 1, dans lequel on ajoute 0,05-0,5% de glucose et 1-10 mg/l de SO₂ audit milieu de culture et l'on ajuste son pH à 3,1-4,5.

4. Procédé selon la revendication 1, dans lequel on choisit ladite souche de bactérie malolactique parmi des souches de Leuconostoc oenos, notamment parmi les souches de Leuconostoc oenos CNCM I-1063, I-1064, I-1065 et I-1066.

5. Procédé selon la revendication 1, dans lequel on déshydrate ladite biomasse, notamment par lyophilisation.

6. Procédé selon la revendication 1, dans lequel on ajoute un agent cryoprotecteur à ladite biomasse, on ajuste son pH à 4,2-4,8 et on la congèle.

7. Levain malolactique obtenu par le procédé selon l'une des revendications 1 à 6 et contenant 5.10⁸-10¹¹ cellules/g.

8. Utilisation du levain malolactique selon la revendication 7 pour la conversion de l'acide malique en acide lactique au cours de la préparation d'un vin.

9. Utilisation selon la revendication 8 dans un procédé de préparation d'un vin léger comprenant une séparation d'une partie du sucre d'un moût de raisin, une fermentation alcoolique du moût appauvri en sucre et une fermentation malolactique du vin à faible teneur en alcool ainsi obtenu.

10. Utilisation selon la revendication 8 dans un procédé de préparation d'un vin léger comprenant une fermentation alcoolique au moins partielle d'un moût de raisin, une élimination d'une partie au moins de l'alcool produit durant cette fermentation alcoolique, et une fermentation malolactique et/ou alcoolique secondaire du vin à faible teneur en alcool ainsi obtenu.
